# FASCICULE DE BREVET EUROPEEN

(11) **EP 3 585 268 B1**
(45) Date de publication et mention de la délivrance du brevet: **28.05.2025**
(21) Numéro de dépôt: 18709679.7
(22) Date de dépôt: 21.02.2018
(51) Int. Cl.: A61B 8/00, A61B 8/10, A61B 8/08

(54) **PROCÉDÉ D'ÉCHOGRAPHIE OCULAIRE A TRANSDUCTEURS ANNULAIRES**
VERFAHREN FÜR AUGENULTRASCHALL MIT RINGFÖRMIGEN WANDLERN
METHOD FOR OCULAR ULTRASOUND WITH ANNULAR TRANSDUCERS

(30) Priorité: 22.02.2017 FR 1751386
(43) Date de publication de la demande: 01.01.2020
(73) Titulaire: Quantel Medical, 63800 Cournon-d'Auvergne (FR)
(72) Inventeur: CHABRIER, Christian, 63670 La Roche Blanche (FR); VENUAT, Cédric, 63100 Clermont Ferrand (FR)
(74) Mandataire: Regimbeau
(86) Numéro de dépôt international: PCT/FR2018/050405
(87) Numéro de publication internationale: WO 2018/154233

(56) Documents cités:
- US-A- 5 092 336
- US-A1- 2005 251 043
- US-A1- 2016 022 490

## Description

### DOMAINE TECHNIQUE DE L'INVENTION

La présente invention appartient au domaine de l'ophtalmologie. Plus précisément, l'invention concerne un procédé d'échographie ophtalmique au moyen de transducteurs annulaires.

L'échographie ophtalmique consiste en l'acquisition d'image d'un œil au moyen d'une sonde ultrasonore émettant un faisceau d'ultrasons. La sonde ultrasonore est positionnée à proximité d'un œil, typiquement en étant placée au contact de l'œil et émet des ultrasons se propageant dans l'œil. Ces ultrasons traversent les structures internes de l'œil, telles que le cristallin, le corps vitré ou la rétine, et sont en partie réfléchis par ces structures. Les ultrasons réfléchis sont captés et enregistrés par la sonde pour donner des images de l'œil. La propagation des ondes ultrasonores dépend non seulement de leur fréquence, mais beaucoup de la configuration spatiale de la sonde ultrasonore qui les émet.

L'acquisition d'images ultrasonores exploitables pour la plus grande part d'un œil obéit à de nombreuses contraintes avec les transducteurs traditionnels. Par exemple, pour examiner un œil complet et son orbite avec une sonde ultrasonore mécaniquement oscillante et obtenir la meilleure image possible, il est nécessaire d'émettre des ondes dans des fréquences allant de 10 à 25 Mhz, afin de pouvoir atteindre une profondeur de plus de 45mm dans l'œil (entre 45 et 60 mm). Il faut également faire varier l'angle d'émission entre 45 à 60°, de focaliser le transducteur à environ 25 mm (entre 18 et 27 mm) juste avant ou sur la rétine (qui constitue la cible privilégiée), et s'assurer une fréquence d'image de 8hz minimum (entre 8 et 16hz) pour visualiser les mouvements du vitrée.

En utilisant un transducteur mono-élément courbe d'un diamètre de 9 mm (diamètre maximal pour avoir 50°de débattement), on peut par exemple obtenir à la distance focale naturelle donné par la courbure du transducteur, à une fréquence d'ultrasons de 20 MHz (avec une bande passante de 10 à 30 Mhz) une résolution totale longitudinal de 75 µm, et une résolution latérale totale possible de 200 µm. Toutefois, l'image devient rapidement floue avec la profondeur puisque la profondeur de champ à 6 dB n'est alors que de 2,5 mm, ce qui signifie que seule une petite zone autour de la rétine présentera un rapport signal sur bruit et une résolution optimum. Par ailleurs, conformément au théorème de Shannon, il est nécessaire d'échantillonner d'au minimum 2 fois la fréquence la plus haute de la bande passante à traiter (30 MHz pour un transducteur de fréquence central de 20Mhz), soit à 60 MHz et de générer suffisamment de ligne afin d'être au moins à 2,5 fois la résolution latérale totale à la distance focale soit 360 lignes minimum pour 50°.

Concernant l'émission ultrasonore elle-même, il est nécessaire d'attendre la fin du signal écho (i.e. les ultrasons réfléchis) d'une ligne échographique pour en renvoyer une autre, afin d'éviter les échos parasites d'une ligne sur l'autre. On a alors une ligne échographique au mieux toute les 90 µs pour une profondeur de 60mm, ce qui se traduit par une vitesse de sonde maximum de 13hz environ. Toutefois, en acceptant certains compromis sur la qualité des images échographiques acquises, il est possible d'arriver à des fréquences d'image allant jusqu'à 20 Hz.

Afin de permettre une résolution et une pénétration dans l'œil supérieure, il a été proposé d'utiliser un transducteur annulaire, c'est-à-dire un transducteur comprenant une pluralité d'éléments transducteurs organisés en anneaux concentriques formant des anneaux transducteurs. L'utilisation d'un transducteur annulaire permet d'augmenter la profondeur de champs pour avoir une image globale de l'œil la meilleure possible quelle que soit sa géométrie (petit, grand, ovale...).

En reprenant la configuration de l'exemple de transducteur mono-élément évoqué plus haut (ondes ultrasonores à 20 MHz et un diamètre de 9 mm), on peut obtenir avec un transducteur à cinq anneaux ultrasonores courbe une profondeur de champ à 6 dB de 18 mm, soit une amélioration d'un facteur 7 environ. L'utilisation d'un transducteur de ce type améliore donc considérablement la qualité de l'image vitréenne et rétinienne de l'œil. De plus, il améliore également le rapport signal sur bruit sur toute la profondeur de champs.

Par ailleurs, des ondes ultrasonores à des fréquences plus élevées allant de 35 à 50 MHz peuvent être utilisées pour augmenter la précision lors d'un examen du pôle antérieur global d'un œil. Un transducteur mono-élément courbe classique, focalisé à une profondeur de 10 mm environ, permet alors d'examiner jusqu'à 16 mm de profondeur. La profondeur de champ obtenue est alors d'environ 1 mm, ce qui est très peu satisfaisant. En utilisant un transducteur annulaire à cinq anneaux, on arrive à une profondeur de champs de 8 mm ce qui rend l'image meilleure dans sa globalité et l'utilisation de cette configuration beaucoup plus simple du fait de sa moindre sensibilité à l'ajustement en profondeur qui doit être réalisé par l'opérateur.

Utiliser plusieurs anneaux concentriques requiert cependant de coordonner émission et réception des signaux de mesure entre ces anneaux. Deux approches ont jusqu'ici été employées.

Une première approche s'apparente au fonctionnement des transducteurs à commande de phase (ou "phased array" en anglais). On émet sur l'ensemble des anneaux transducteurs avec un délai d'émission calculé entre chaque anneau transducteur pour que les ondes ultrasonores échographiques arrivent en phase à une certaine profondeur. On additionne ensuite en temps réel les lignes échographiques obtenues par la réception des échos ultrasonores par l'ensemble des anneaux transducteurs. On obtient ainsi une image de grande qualité autour de la profondeur visée, et la rapidité est comparable à celle d'un transducteur mono-élément.

Par exemple, la demande de brevet US 2005/251043 A1 décrit un procédé d'exploration et de visualisation de tissus d'origine humaine ou animale, dans lequel :
- on positionne une sonde ultrasonore portée par une tête pilotée par l'intermédiaire d'un système de positionnement dans les trois dimensions, notamment commandé par un ordinateur au droit de ladite structure de tissus,
- on commande la sonde de manière à ce qu'elle génère des faisceaux d'ondes convergentes ultrasonores de haute fréquence (de l'ordre de 30 à 50 MHz), ces ondes étant focalisées au niveau d'une zone donnée de structure de tissus, selon une distance de pénétration comprise entre 20 et 30 mm,
- on effectue un balayage de la structure de tissus par le système de positionnement piloté par l'ordinateur, en effectuant parallèlement une acquisition, par l'ordinateur, des signaux réfléchis par la structure de tissus,
- on effectue divers traitements de signal sur les données issues du balayage, pour améliorer la restitution des informations et faciliter l'interprétation par le praticien.

Dans cette demande de brevet US 2005/251043 A1, on utilise une sonde à focalisation dynamique réalisée par un procédé de commande électronique ou numérique, composée d'une sonde multiéléments, à symétrie circulaire, composée de plusieurs transducteurs annulaires concentriques régulièrement espacés sur une surface plane ou à concavité sphérique. Ces transducteurs sont indépendants les uns des autres et sont commandés individuellement à l'émission et à la réception par des impulsions décalées dans le temps. En particulier, une focalisation dynamique est obtenue en introduisant un déphasage-retard temporel-à l'émission entre les différents anneaux. L'ensemble des anneaux transducteurs émettent avec un délai d'émission calculé entre chaque anneau transducteur pour que les ondes ultrasonores échographiques arrivent en phase à une certaine profondeur. On additionne ensuite en temps réel les lignes échographiques obtenues par la réception des échos ultrasonores par l'ensemble des anneaux transducteurs.

Toutefois, lorsqu'il est souhaitable d'obtenir une image globale de qualité d'un œil, plusieurs profondeurs doivent être ciblées. Il est donc nécessaire de faire plusieurs passages en modifiant les délais d'émission affectant chaque anneau transducteur pour atteindre différentes profondeurs. Il en résulte un temps d'acquisition très long puisque la fréquence d'acquisition se voit divisée par le nombre de profondeurs différentes à analyser pour reconstituer l'image globale.

Une seconde approche s'apparente au fonctionnement des radars. Un seul anneau transducteur est excité et émet des ondes ultrasonores. En revanche, les signaux de tous les anneaux transducteurs, résultant de la réception par ces anneaux transducteurs des ondes ultrasonores réfléchies, sont récupérés et recalés (pour compenser la différence de marche entre anneaux transducteurs). Afin d'obtenir l'image finale, il est toutefois nécessaire d'utiliser tous les anneaux transducteurs en émission, ce qui nécessite donc autant d'itérations émission-réception que le nombre d'anneaux transducteurs. Ainsi, dans un exemple avec cinq anneaux transducteurs, cela signifie de procéder à cinq émissions successives, avec cinq réceptions par émissions, soit un total de 25 lignes échographiques partielles qui doivent être traitées pour donner une ligne échographique globale. Par conséquent, cette approche requiert cinq fois plus de temps qu'avec un transducteur mono-élément. Or, la rapidité est importante en échographie ophtalmique afin de pouvoir observer les mouvements du corps vitré.

Par ailleurs, cette approche demande un post-traitement important en raison des nombreuses lignes échographiques partielles, ce qui implique un temps de calcul non négligeable, pouvant nécessiter une gestion des décalages par tranche. De plus, chaque émission ne se faisant que sur un anneau transducteur, le résultat final du rapport signal sur bruit à la distance focale est similaire à celui d'un transducteur mono-élément mais inférieur de 6 dB à la méthode précédente. La demande de brevet US 2013/0093901 A1 emploie cette approche, et afin d'accélérer l'acquisition d'image, propose de ne pas employer certaines lignes, ce qui conduit à une moindre qualité d'image en résolution, sensibilité et pénétration.

### PRESENTATION DE L'INVENTION

L'invention a pour but de proposer un procédé d'échographie oculaire utilisant une sonde ultrasonore à plusieurs anneaux transducteurs permettant d'acquérir des images de bonne qualité et rapidement.

A cet effet, il est proposé un procédé d'échographie oculaire utilisant une sonde ultrasonore selon la revendication 1.

Le procédé est avantageusement complété par les caractéristiques suivantes, prises seules ou en une quelconque de leur combinaison techniquement possible :
- dans chaque cycle, les itérations sont effectuées dans le même ordre ;
- le signal de mesure d'un anneau transducteur résulte de la numérisation du signal de réception généré par cet anneau transducteur lors de la réception par ledit anneau transducteur d'ondes ultrasonores réfléchies résultant de l'émission d'ondes ultrasonores par un groupe d'anneaux transducteurs, un signal de mesure se définissant comme une suite chronologique de points discrets auxquels sont associées des valeurs correspondantes, et la combinaison de signaux de mesure pour donner une ligne échographique lors d'une itération consiste à additionner les valeurs associées à des points discrets synchrones desdits signaux de mesure;
- la combinaison de signaux de mesure pour donner une ligne échographique lors d'une itération est restreinte à une sélection de points discrets, lesdits points discrets étant sélectionnés de sorte à opérer un décalage chronologique entre les signaux de mesure compensant les différences de chemin acoustique résultant de la géométrie du transducteur, le synchronisme des points discrets prenant en compte ce décalage ;
- des points discrets sont ajoutés au signal de mesure en faisant la convolution dudit signal de mesure avec un sinus cardinal glissant de sorte qu'une période entre les points discrets soit inférieure à l'inverse d'au moins dix fois la fréquence d'émission ;
- une ligne échographique se définit comme une suite chronologique de points discrets auxquels sont associées des valeurs correspondantes, et dans lequel la combinaison de lignes échographiques pour donner une ligne affichable consiste à additionner les valeurs associées à des points discrets synchrones desdites lignes échographiques ;
- les anneaux transducteurs sont regroupés en k groupes d'anneaux regroupant chacun entre 2 et n-1 anneaux transducteurs, avec 1<k<n;
- n= 5, la sonde ultrasonore comprenant cinq anneaux transducteurs.

L'invention concerne également un produit programme d'ordinateur comprenant des instructions de code de programme enregistrées sur un support non-volatile utilisable dans un ordinateur pour l'exécution d'étapes de traitement du procédé selon l'invention, lorsque ledit programme est exécuté sur un ordinateur.

L'invention concerne également un système d'échographie comprenant une sonde ultrasonore selon la revendication 10.

### PRESENTATION DES FIGURES

L'invention sera mieux comprise, grâce à la description ci-après, qui se rapporte à des modes de réalisations et des variantes selon la présente invention, donnés à titre d'exemples non limitatifs et expliqués avec référence aux dessins schématiques annexés, dans lesquels :
- la figure 1 illustre schématiquement la configuration annulaire des éléments transducteur d'une sonde ultrasonore selon un mode de réalisation possible de l'invention,
- les figures 2, 3 et 4 illustrent schématiquement le déroulement d'exemples de procédé d'échographie selon différents modes de réalisation de l'invention.

### DESCRIPTION DETAILLEE

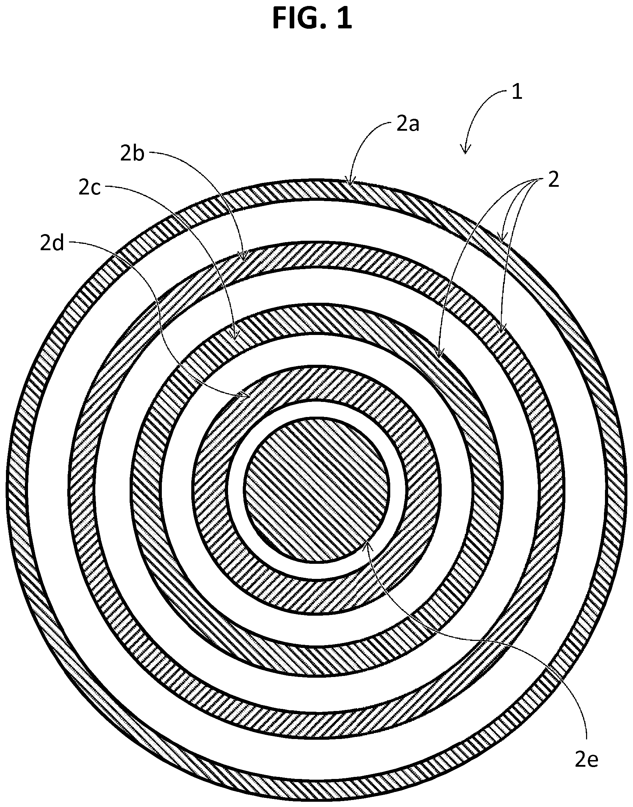

En référence à la figure 1, il est utilisé une sonde ultrasonore 1 comportant une pluralité d'éléments transducteurs organisés en n anneaux concentriques 2 formant n anneaux transducteurs. Dans l'exemple de la figure 1, il y a cinq anneaux transducteurs, désignés respectivement depuis l'extérieur vers le centre par 2a, 2b, 2c, 2d et 2e, et par conséquent n = 5. Il est bien entendu cependant que n peut prendre d'autres valeurs. Toutefois, le nombre d'anneaux transducteurs 2 est supérieur à trois (i.e. n>3), et de préférence supérieur à quatre (i.e. n>4). Les éléments transducteurs sont par exemple piézoélectriques, configurés pour émettre des ultrasons se propageant dans l'oeil. Ces ultrasons ont typiquement une fréquence comprise entre 10 et 100 MHz. Il est à noter ici que l'anneau transducteur central 2e est plein, et constitue de ce fait un disque transducteur. L'anneau transducteur central 2e pourrait aussi bien être creux, c'est-à-dire avec un centre vide comme les autres anneaux transducteurs périphériques 2a, 2b, 2c et 2d. Les différents anneaux transducteurs étant concentriques, il est nécessaire que les anneaux transducteurs périphériques 2a, 2b, 2c et 2d soient creux pour qu'ils puissent être imbriqués les uns dans les autres. Il n'y a pas cette nécessité l'anneau transducteur central 2e, qui peut donc être plein.

La focale naturelle de la sonde ultrasonore 1 est donnée par la courbure des éléments transducteurs ou par l'ajout de lentille en regard de sa face d'émission. Pour l'examen oculaire cette courbure peut varier de plat à un rayon de courbure de 9 mm par exemple. Le plus grand anneau transducteur 2a présente un diamètre extérieur compris entre 3 et 10 mm, par exemple 9mm, et présente une largeur de 0,05 mm. Le plus petit anneau transducteur 2e présente un diamètre extérieur compris entre 0,1 et 0,3 mm par exemple 0,2 mm. Les anneaux transducteurs 2 sont séparés par une distance comprise entre 0,02 et 0,1 mm par exemple 0,05 mm. De préférence, afin que les anneaux transducteurs présentent entre eux une puissance équivalente, on peut chercher à ce que leurs surfaces respectives soient de tailles similaires, et idéalement identiques. A cet effet, la largeur des anneaux transducteurs décroît de préférence avec leur distance au centre commun.

Pour la mise en œuvre du procédé d'échographie ophtalmique, la sonde ultrasonore 1 est positionnée par rapport à un œil, afin de pouvoir émettre et recevoir des ondes ultrasonores se propageant à l'intérieur de cet œil. La sonde ultrasonore 1 peut être mise au contact de l'œil, c'est-à-dire accolée à la cornée ou à la sclérotique, éventuellement recouverte d'un gel. On peut également prévoir la présence d'une poche de liquide tel que de l'eau entre la sonde ultrasonore 1 et l'œil, cette proche pouvant typiquement être formée par une membrane fermée définitivement sur la sonde ultrasonore 1. La sonde ultrasonore 1 peut également être immergée dans un liquide contenu dans une cupule ouverte contre l'œil, le liquide servant de milieu de propagation intermédiaire entre la sonde ultrasonore 1 et l'œil.

Une fois positionnée, la sonde ultrasonore 1 est commandée pour émettre et recevoir des ondes ultrasonores. Chaque anneau transducteur 2 est commandé individuellement, et en réponse à une excitation (un signal de tension électrique), un anneau transducteur 2 émet des ondes ultrasonores à une fréquence d'émission. La fréquence d'émission est comprise entre 10 et 100 MHz. Dans l'exemple qui suit, une fréquence d'émission de 20 MHz sera décrite.

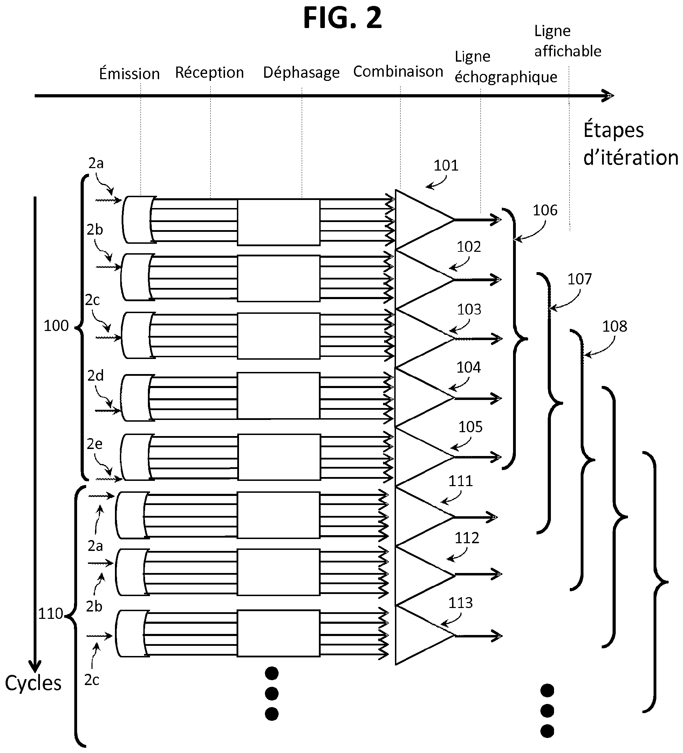

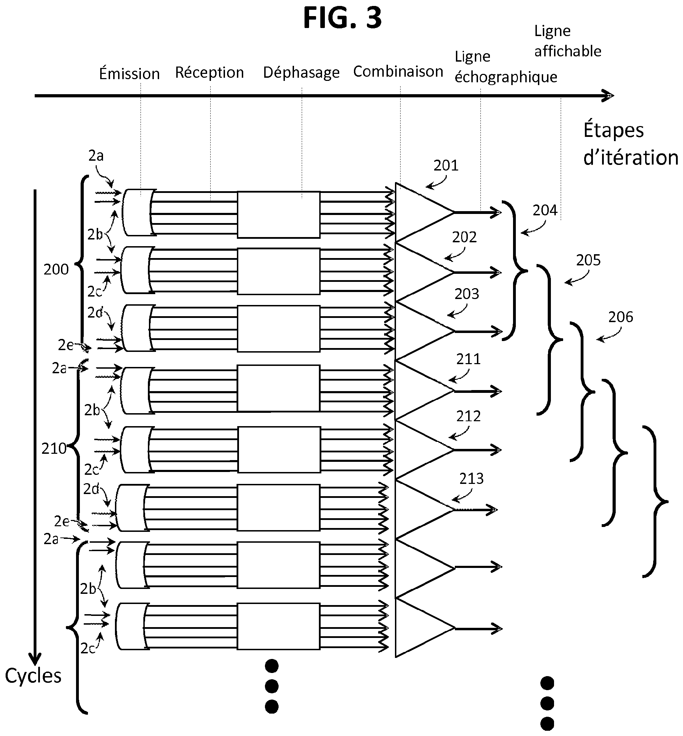

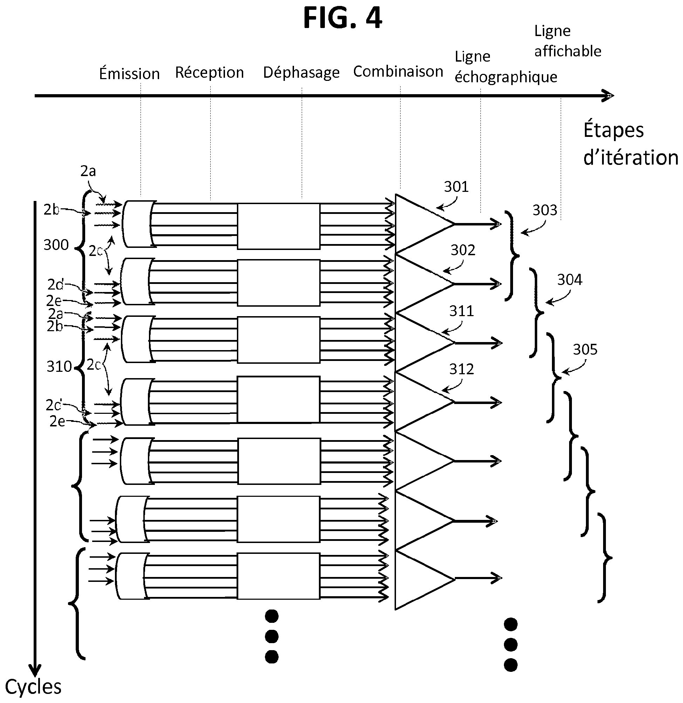

Dans le cadre du procédé, les anneaux transducteurs 2 sont regroupés en plusieurs groupes d'anneaux regroupant entre 1 et n-1 anneaux transducteurs. Par exemple, si n=5, alors chaque groupe d'anneaux peut regrouper un, deux, trois ou quatre anneaux transducteurs 2. Le nombre de groupes d'anneaux est de k, avec 1≤k≤n. De préférence, k est au moins égal à deux, et de préférence 1<k≤n. De préférence également, 1<k<n. Les anneaux transducteurs sont par conséquent regroupés en plusieurs groupes d'anneaux (k≥2). Ainsi, dans l'exemple de la figure 2, n=5 et k=5, dans l'exemple de la figure 3, n=5 et k=3, dans l'exemple de la figure 4, n=5 et k=2.

Afin d'assurer l'équivalence entre les groupes, les différents groupes d'anneaux transducteurs présentent de préférence le même nombre d'anneaux transducteurs 2. Un anneau transducteur 2 peut faire partie de deux groupes d'anneaux. Toutefois, chaque groupe d'anneaux transducteurs se différencie d'un autre groupe d'anneaux transducteurs par au moins un anneau transducteur 2 différent des anneaux transducteurs 2 dudit autre groupe d'anneaux transducteurs. De préférence, chaque groupe d'anneaux transducteurs comprend au moins un anneau transducteur 2 n'appartenant à aucun autre groupe d'anneaux transducteurs.

Par exemple, dans le cas de la figure 3, un premier groupe d'anneaux transducteurs regroupe les anneaux transducteurs 2a et 2b, un deuxième groupe d'anneaux transducteurs regroupe les anneaux transducteurs 2b et 2c, et le troisième groupe d'anneaux transducteurs regroupe les anneaux transducteurs 2d et 2e. On constate ainsi que l'anneau transducteur 2b fait partie de deux groupes d'anneaux transducteurs, mais également que l'anneau transducteur 2a fait partie du seul premier groupe d'anneaux et d'aucun autre groupe d'anneaux, que l'anneau transducteur 2c fait partie du seul troisième groupe d'anneaux et d'aucun autre groupe d'anneaux, et les anneaux transducteurs 2c et 2d font partie du seul troisième groupe et d'aucun autre groupe d'anneaux.

Ces regroupements en différents groupe se traduisent par une commande d'émission commune aux anneaux transducteurs 2 d'un même groupe d'anneaux à un instant d'émission. Il est possible de modifier le nombre ou la composition des groupes d'anneaux transducteurs d'une mise en application du procédé à une autre. Il est à noter qu'il n'est pas nécessaire de sélectionner tous les anneaux transducteurs 2 de la sonde ultrasonore 1 pour émettre des ultrasons. Dans certaines configurations, des anneaux transducteurs 2 peuvent ne pas émettre des ultrasons et n'être utilisés qu'en réception.

Le procédé comprend une pluralité de cycles. Un cycle comprend plusieurs itérations d'émission-réception d'ondes ultrasonores faisant intervenir à chaque fois un groupe d'anneaux transducteurs différent. Chaque cycle parcoure l'ensemble des k groupes d'anneaux transducteurs. Lors de chaque itération :
- un groupe d'anneaux transducteurs est excité de sorte que seuls les anneaux transducteurs 2 dudit groupe d'anneaux transducteurs émettent des ondes ultrasonores à une fréquence d'émission, tandis que les autres anneaux transducteurs 2 ne faisant pas partie dudit groupe d'anneaux transducteurs n'émettent pas d'ondes ultrasonores à la fréquence d'émission, et
- les n signaux de mesure de l'ensemble des n anneaux transducteurs sont récupérés, chaque signal de mesure résultant de la réception par un anneau transducteur 2 d'ondes ultrasonores réfléchies résultant de l'émission d'ondes ultrasonores par les anneaux transducteurs ou le groupe d'anneaux transducteurs,
- les n signaux de mesure sont combinés pour donner une ligne échographique.

De préférence, dans chaque cycle, les itérations sont effectuées dans le même ordre. Les itérations de chaque cycle permettent de déterminer et d'afficher une ligne affichable. Il y a donc au moins autant d'itérations de cycles que de lignes dans l'image affichée. Toutefois, entre chaque cycle, chaque image ou groupe d'images, les modalités d'émission des ondes ultrasonores peuvent être modifiées pour privilégier la résolution (émissions individuelles), la pénétration (émission sur anneaux groupés) ou la vitesse (non utilisation de tous les anneaux). Sachant que toutes les combinaisons sont possibles sur chaque image et éventuellement sur chaque ligne en termes d'émission, de groupement d'anneaux et de nombre d'anneaux utilisés en émission ou en réception.

### Emission

Dans l'exemple de la figure 2, chaque groupe d'anneaux de transducteurs ne comprend qu'un unique anneau transducteur 2 ultrasonore. Le premier groupe d'anneaux est constitué de l'anneau transducteur ultrasonore 2a, le deuxième groupe d'anneaux est constitué de l'anneau transducteur ultrasonore 2b, le troisième groupe d'anneaux est constitué de l'anneau transducteur ultrasonore 2c, le quatrième groupe d'anneaux est constitué de l'anneau transducteur ultrasonore 2d, et le cinquième groupe d'anneaux est constitué de l'anneau transducteur ultrasonore 2e. Il y a donc cinq groupes d'anneaux (k=5) pour cinq anneaux transducteurs (n=5).

Un premier cycle 100 comprend cinq itérations 101, 102, 103, 104 et 105. Dans une première itération 101, seul l'anneau transducteur 2a du premier groupe d'anneaux est excité par un signal électrique de commande et émet des ondes ultrasonores. Dans une deuxième itération 102, seul l'anneau transducteur 2b du deuxième groupe d'anneaux est excité par un signal électrique de commande et émet des ondes ultrasonores. Dans une troisième itération 103, seul l'anneau transducteur 2c du troisième groupe d'anneaux est excité par un signal électrique de commande et émet des ondes ultrasonores. Dans une quatrième itération 104, seul l'anneau transducteur 2d du quatrième groupe d'anneaux est excité par un signal électrique de commande et émet des ondes ultrasonores. Dans une cinquième itération 105, seul l'anneau transducteur 2e du cinquième groupe d'anneaux est excité par un signal électrique de commande et émet des ondes ultrasonores.

Une fois ce premier cycle 100 terminé, c'est-à-dire lorsque ledit premier cycle 100 a parcouru l'ensemble des cinq groupes d'anneaux transducteurs 101, 102, 103, 104, 105, un deuxième cycle 110 commence alors, parcourant l'ensemble des cinq groupes d'anneaux transducteurs au cours de cinq itérations 111, 112, 113 de la même façon que premier cycle 100.

Dans l'exemple de la figure 3, chaque groupe d'anneaux de transducteurs est constitué de deux anneaux transducteurs ultrasonores. Le premier groupe d'anneaux est constitué par les anneaux transducteurs ultrasonores 2a et 2b, le deuxième groupe d'anneaux est constitué par les anneaux transducteurs ultrasonores 2b et 2c, le troisième groupe d'anneaux est constitué par les anneaux transducteurs ultrasonores 2d et 2e. Il y a donc trois groupes d'anneaux (k=3) pour cinq anneaux transducteurs (n=5).

Un premier cycle 200 comprend trois itérations 201, 202, 203. Dans une première itération 201, seuls les anneaux transducteurs 2a et 2b du premier groupe d'anneaux sont excités par un signal électrique de commande et émettent des ondes ultrasonores. Dans une deuxième itération 202, seuls les anneaux transducteurs 2b et 2c du deuxième groupe d'anneaux sont excités par un signal électrique de commande et émettent des ondes ultrasonores. Dans une troisième itération 203, seuls les anneaux transducteurs 2d et 2e du troisième groupe d'anneaux sont excités par un signal électrique de commande et émettent des ondes ultrasonores. Une fois ce premier cycle 200 terminé, c'est-à-dire lorsque ledit premier cycle 200 a parcouru l'ensemble des trois groupes d'anneaux transducteurs, un deuxième cycle 210 commence alors, parcourant l'ensemble des trois groupes d'anneaux transducteurs au cours de trois itérations 211, 212, 213 de la même façon que premier cycle 200.

Dans l'exemple de la figure 4, il y a deux groupes d'anneaux transducteurs, et chaque groupe d'anneaux transducteurs est constitué de trois anneaux transducteurs. Le premier groupe est constitué par les anneaux transducteurs ultrasonores 2a, 2b, et 2c, le deuxième groupe est constitué par les anneaux transducteurs ultrasonores 2c, 2d, et 2e. Il y a donc deux groupes d'anneaux (k=2) pour cinq anneaux transducteurs (n=5).

Un premier cycle 300 comprend deux itérations 301, 302. Dans une première itération 301, seuls les trois anneaux transducteurs 2a, 2b, et 2c du premier groupe d'anneaux sont excités par un signal électrique de commande et émettent des ondes ultrasonores. Dans une deuxième itération 302, seuls les trois anneaux transducteurs 2c, 2d, et 2e du deuxième groupe d'anneaux sont excités par un signal électrique de commande et émettent des ondes ultrasonores. Une fois ce premier cycle 300 terminé, c'est-à-dire lorsque ledit premier cycle 300 a parcouru l'ensemble des deux groupes d'anneaux transducteurs 301, 302, un deuxième cycle 310 commence alors, parcourant l'ensemble des deux groupes d'anneaux transducteurs au cours de deux itérations 311, 312, de la même façon que premier cycle 300.

Pour chaque itération, on a donc une émission d'ondes ultrasonores, une réception d'ondes ultrasonores, et la combinaison des signaux de mesure résultant de cette réception.

### Réception

Les ultrasons émis se propagent dans l'oeil, traversent les structures internes de l'oeil, telles que le cristallin, le corps vitré ou la rétine, et sont en partie réfléchis par ces structures et renvoyés vers la sonde ultrasonore 1. La réception, par un anneau transducteur 2, d'ondes ultrasonores réfléchies engendre un signal de mesure. Tous les anneaux transducteurs 2 reçoivent ces ondes ultrasonores réfléchies et génèrent des signaux de mesures. Ainsi, dans le cas où la sonde ultrasonore comprend cinq anneaux transducteurs 2, cinq signaux de mesure sont générés et sont utilisés.

Plus précisément, la réception des ondes ultrasonores par un anneau transducteur 2 provoque l'apparition d'un signal de réception, électrique et analogique, en sortie dudit anneau transducteur 2. Ce signal de réception est alors numérisé pour donner un signal de mesure. Un signal de mesure est donc un signal numérique et se définit comme une suite chronologique de points discrets auxquels sont associées des valeurs correspondantes, déterminées à partir du signal de réception.

La numérisation des signaux de mesure peut être faite à une fréquence telle que le pas de numérisation soit suffisamment fin pour effectuer ensuite un décalage compensant les différences de chemin des ondes ultrasonores pour les différents signaux de mesure. Cette fréquence de numérisation devrait alors être d'au moins 10 fois la fréquence d'émission, soit par exemple 200 MHz pour une fréquence d'émission de 20 MHz, de préférence d'au moins 12 à 15 fois la fréquence d'émission afin de traiter toute la bande passante du signal en réception, qui va souvent jusqu'à 1,5 fois la fréquence d'émission.

Ces fréquences de numérisation élevées peuvent conduire à des numériseurs compliqués surtout pour des transducteurs utilisant des fréquences hautes, telles que 50 Mhz ou plus. C'est pour éviter ces problèmes qu'alternativement, il est également possible d'ajouter des points discrets à chaque signal de mesure en faisant la convolution dudit signal de mesure avec un sinus cardinal glissant de sorte qu'une période entre les points discrets soit inférieure à l'inverse d'au moins dix fois la fréquence d'émission, et de préférence inférieure à 12 à 15 fois la fréquence d'émission.

Une fois les signaux de mesure obtenus soit par numérisation directe soit en ajoutant des points supplémentaires, on peut choisir sur chaque signal de mesure une sélection de points discrets pour restreindre le signal de mesure à ces points discrets sélectionnés. Les points discrets sont sélectionnés de sorte à opérer un décalage chronologique entre les différents signaux de mesure compensant les différences de chemin acoustique résultant de la géométrie des anneaux transducteurs 2, le synchronisme des points discrets prenant en compte ce décalage.

En effet, les ondes ultrasonores sont émises et reçues par différents anneaux transducteurs 2 disposés à différentes positions. Il en résulte des différences de chemin acoustique se traduisant par des décalages temporels. A titre d'exemple, le tableau 1 ci-dessous montre le retard absolu en nanosecondes affectant les ondes ultrasonores lors de leur trajet par un point de focalisation à 15 mm de profondeur dans l'axe de leur point central en fonction des anneaux en émission et en réception pour une fréquence d'émission à 20 Mhz et une sonde ultrasonore de 9 mm de diamètre (diamètre de l'anneau transducteur extérieur 2a) focalisé naturellement à 22 mm:

**Tableau 1**

| | Anneau en réception | | | | |
|---|---|---|---|---|---|
| Anneau en émission | 2e | 2d | 2c | 2b | 2a |
| anneau 2e | 25 | 53 | 82 | 112 | 143 |
| anneau 2d | 53 | 81 | 109 | 140 | 171 |
| anneau 2c | 82 | 109 | 139 | 169 | 200 |
| anneau 2b | 112 | 140 | 169 | 199 | 230 |
| anneau 2a | 143 | 171 | 200 | 230 | 261 |

Bien évidemment, le retard est d'autant plus important que l'anneau transducteur 2 est éloigné de leur centre commun, et l'anneau transducteur extérieur 2a est le plus affecté. Le retard affectant les ondes ultrasonores se traduit par des décalages temporels entre les signaux de mesure des différents anneaux transducteurs.

C'est ce décalage temporel entre les signaux de mesure entre les anneaux transducteurs 2 qui importe pour pouvoir exploiter les signaux de mesure en provenance de différents anneaux transducteurs 2. En reprenant l'exemple ci-dessus et en prenant comme référence le signal de mesure du transducteur annulaire central 2e pour une émission par ce transducteur annulaire central 2e, les décalages temporels affectant les autres signaux de mesure sont donnés par le tableau 2:

**Tableau 2**

| | Anneau en réception | | | | |
|---|---|---|---|---|---|
| Anneau en émission | 2e | 2d | 2c | 2b | 2a |
| anneau 2e | 0 | 28 | 57 | 87 | 118 |
| anneau 2d | 28 | 56 | 84 | 115 | 146 |
| anneau 2c | 57 | 84 | 114 | 144 | 175 |
| anneau 2b | 87 | 115 | 144 | 174 | 205 |
| anneau 2a | 118 | 146 | 175 | 205 | 236 |

Ce décalage temporel entre les signaux de mesure se traduit, après numérisation, par un décalage en nombre de points discrets. Ainsi, en reprenant l'exemple ci-dessus, les décalages en point pour une numérisation avec un pas de 2 ns est donnée par le tableau 3:

**Tableau 3**

| | Anneau en réception | | | | |
|---|---|---|---|---|---|
| Anneau en émission | 2e | 2d | 2c | 2b | 2a |
| anneau 2e | 0 | 14 | 29 | 44 | 59 |
| anneau 2d | 14 | 28 | 42 | 58 | 73 |
| anneau 2c | 29 | 42 | 57 | 72 | 88 |
| anneau 2b | 44 | 58 | 72 | 87 | 103 |
| anneau 2a | 59 | 73 | 88 | 103 | 118 |

Il faut donc prendre en compte ce décalage en point des signaux de mesure pour faire correspondre les informations qui y sont contenues, ce qui peut être fait simplement par le biais de la sélection des points discrets de chaque signal de mesure. Par exemple, xₜ₁ est un point discret d'un signal de mesure d'un premier anneau transducteur 2 correspondant à l'instant t₁. yₜ₁ est un point discret d'un signal de mesure d'un second anneau transducteur 2 correspondant à l'instant t₁. Toutefois, xₜ₁ et yₜ₁ ne rendent pas compte des mêmes ondes ultrasonores. En effet, en raison de leur disposition sur la sonde ultrasonore, les ondes sonores arrivant sur le second anneau transducteur 2 ont un plus long chemin à parcourir et arrivent avec un retard d par rapport à leur arrivée sur le premier anneau transducteur 2. Par conséquent, c'est le point y_{t1+d} qui correspond aux mêmes ondes ultrasonores que le point xt₁. Si on sélectionne pour le premier signal de mesure les points xₜ₁, xₜ₂, xₜ₃, ..., on sélectionne pour le second signal de mesure les points y_{t1+d}, x_{t2+d}, x_{t3+d}, .... On peut ainsi prendre en compte le décalage entre les signaux de mesure de façon simplifiée, sans être obligé de mettre en oeuvre un recalage temporel des signaux de mesure. Cette simplicité permet de mettre en oeuvre cette prise en compte du décalage pratiquement en temps réel.

### Combinaison des signaux de mesure

Les signaux de mesure des différents anneaux transducteurs sont ensuite combinés pour donner une ligne échographique. Cette ligne échographique est représentative de la réponse des anneaux transducteurs 2 à l'émission d'ondes ultrasonores par le groupe d'anneaux transducteurs qui les a émis lors de cette itération. La combinaison de signaux de mesure pour donner une ligne échographique lors d'une itération consiste à additionner les valeurs associées à des points discrets desdits signaux de mesure, avec un décalage correspondant au retard respectif affectant chaque signal de mesure. En reprenant l'exemple ci-dessus, on additionne le point discret xₜ₁ d'un signal de mesure d'un premier anneau transducteur 2 avec le point discret y_{t1+d} d'un autre signal de mesure d'un deuxième anneau transducteur 2, les deux points étant synchrones à l'instant t₁ moyennant le retard d affectant le signal de mesure du deuxième anneau transducteur 2 par rapport au signal de mesure du premier anneau transducteur 2. La ligne échographique peut bien entendu subir différents traitements classiques tels que des filtrages, des décalages ou une mise à l'échelle.

Lorsque l'on dispose de suffisamment de points discrets dans chaque signal de mesure, c'est-à-dire avec une fréquence supérieure à au moins 10 fois la fréquence d'émission, on obtient alors qu'au délai théorique affectant un signal de mesure correspond un décalage en nombre de points discrets, comme expliqué plus haut. Les délais n'étant plus alors des temps, mais un décalage dans le choix des points dans les signaux de mesure, il est possible de combiner les signaux de mesure en les additionnant pratiquement en temps réel. La ligne échographique peut bien entendu subir différents traitements classiques tels que des filtrages, des décalages ou une mise l'échelle.

### Combinaison des lignes échographiques

A chaque itération, les étapes ci-dessus sont réitérées, en modifiant cependant le groupe d'anneaux transducteurs 2 émettant les ondes ultrasonores. On obtient donc une ligne échographique à chaque itération. Toutefois, une ligne échographique ne représente la réponse des anneaux transducteurs 2 qu'à l'émission d'ondes ultrasonores par les seuls anneaux transducteurs 2 du groupe d'anneaux ultrasonores intervenant dans l'itération.

Il est donc prévu de combiner les k lignes échographiques résultant des k itérations les plus récentes en une ligne affichable. Les lignes échographiques se définissent comme des suites chronologiques de points discrets auxquels sont associées des valeurs correspondantes, et la combinaison de lignes ultrasonores pour donner une ligne affichable consiste à additionner les valeurs associées à des points discrets synchrones desdites lignes échographiques. Il est à noter que la combinaison de ces lignes échographiques peut être effectuée dès lors que lesdites lignes échographiques sont disponibles. Par conséquent, les combinaisons peuvent être effectuées parallèlement à la poursuite des cycles suivant le premier cycle.

Les k lignes échographiques combinées peuvent résulter des k itérations d'un cycle s'il vient de se terminer, ou des k-i dernières itérations d'un cycle et des i itérations du cycle suivant, avec 1<i<k. Par exemple, en référence à la figure 2, les itérations 101, 102, 103, 104, 105 du premier cycle 100 donnent chacune une ligne échographique (k=5). Une première combinaison 106 porte sur les lignes échographiques résultant des itérations 101, 102, 103, 104, 105 du premier cycle 100. La seconde combinaison 107 porte sur les quatre dernières lignes échographiques du premier cycle 100, c'est-à-dire résultant des itérations 102, 103, 104, 105, et sur la première ligne échographique du deuxième cycle 110, c'est-à-dire résultant de l'itération 111. La troisième combinaison 108 porte sur les trois dernières lignes échographiques du premier cycle 100, c'est-à-dire résultant des itérations 103, 104, 105, et sur les deux premières lignes échographiques du deuxième cycle 110, c'est-à-dire résultant des itérations 111 et 112. Les combinaisons suivantes se déroulent de manière similaire ainsi de suite, en décalant d'une itération pour chaque nouvelle ligne affichable.

Par exemple en référence à la figure 3, les itérations 201, 202, 203 du premier cycle 200 donnent chacune une ligne échographique (k=3). Une première combinaison 204 porte sur les trois lignes échographiques résultant des itérations 201, 202, et 203 du premier cycle 200. La seconde combinaison 205 porte sur les deux dernières lignes échographiques du premier cycle 200, c'est-à-dire résultant des itérations 202 et 203, et sur la première ligne échographique du deuxième cycle 210, c'est-à-dire résultant de l'itération 211. La troisième combinaison 206 porte sur la dernière ligne échographique du premier cycle 200, c'est-à-dire résultant de l'itération 203, et sur les deux premières lignes échographiques du deuxième cycle 210, c'est-à-dire résultant des itérations 211 et 212. Les combinaisons suivantes se déroulent de manière similaire ainsi de suite, en décalant d'une itération pour chaque nouvelle ligne affichable.

Par exemple en référence à la figure 4, les itérations 301 et 302 du premier cycle 300 donnent chacune une ligne échographique (k=2). Une première combinaison 303 porte sur les deux lignes échographiques résultant des itérations 301 et 302 du premier cycle 300. La seconde combinaison 304 porte sur la dernière ligne échographique du premier cycle 300, c'est-à-dire résultant de l'itération 302, et sur la première ligne échographique du deuxième cycle 310, c'est-à-dire résultant de l'itération 311. La troisième combinaison 305 porte sur les deux lignes échographiques résultant des itérations 311 et 312 du deuxième cycle 310. Les combinaisons suivantes se déroulent de manière similaire ainsi de suite, en décalant d'une itération pour chaque nouvelle ligne affichable.

On fait ainsi une combinaison glissante des k dernières lignes échographiques résultant des k itérations les plus récentes pour obtenir chacune des lignes affichables. Pour afficher N lignes affichables sur un écran, on procède alors à N+k-1 itérations. Par exemple, pour afficher 400 lignes affichables, on procède à 404 itérations si k=5, ce qui représente un surcoût en itérations négligeable. Pour rendre les lignes numériques du signal affichables et ainsi produire une image on peut leur fait subir bien entendu différents traitements classiques tel que filtrage, redressement, décalage et mise à l'échelle logarithmique.

Avec cette approche, il est possible de modifier en temps réel les modalités d'émission des ondes ultrasonores, en modifiant la composition des groupes d'anneaux, par exemple en passant de groupes de un anneau transducteur 2 à des groupes de trois anneaux transducteurs 2. Il est également possible de modifier les modalités de traitement des signaux de mesure, en changeant les retards affectant ceux-ci lors de leur combinaison. En fonction de la cible médicale choisie, on peut ainsi favoriser la résolution, la sensibilité, la pénétration ou la vitesse en utilisant des configurations d'émission ou de réception différentes.

Par exemple, en utilisant des groupes d'anneaux constitués d'un unique anneau transducteur 2 comme sur la figure 1, on obtient le meilleur compromis entre la résolution, la pénétration et la sensibilité sur l'image globale d'un oeil.

Avec cette approche, on augmente sensiblement la vitesse par rapport à l'approche traditionnelle à commande de phase, puisqu'à chaque cycle ou itération, on examine toute la profondeur inspectée. Il n'y a donc plus besoin d'effectuer de multiples passages au même endroit pour imager à différentes profondeurs. Par rapport à l'approche type radar, on obtient après le premier cycle une ligne affichable par itération, ce qui permet d'être beaucoup plus rapide puisque l'approche type radar ne produit qu'une ligne affichable par cycle. On arrive ainsi à une vitesse quasi similaire à un mono-transducteur, ce qui est fondamental en ophtalmologie, notamment en raison des rapides mouvements de l'œil.

Une unité de traitement automatisé de données comprenant au moins un processeur et une mémoire est utilisée pour le traitement des données d'images, et notamment pour combiner les signaux de mesure ou les lignes.

L'invention n'est pas limitée au mode de réalisation décrit et représenté aux figures annexées. Des modifications restent possibles, notamment du point de vue de la constitution des divers éléments sans sortir pour autant du domaine de protection de l'invention.

## Revendications

1. Procédé d'échographie oculaire utilisant une sonde ultrasonore (1) comportant une pluralité d'éléments transducteurs organisés en au moins n anneaux concentriques formant n anneaux transducteurs (2, 2a, 2b, 2c, 2d, 2e), dans lequel les anneaux transducteurs sont regroupés en plusieurs groupes d'anneaux regroupant chacun entre 1 et n-1 anneaux transducteurs, chaque groupe d'anneaux se différenciant d'un autre groupe d'anneaux transducteurs par au moins un anneau transducteur différent des anneaux transducteurs dudit autre groupe d'anneaux transducteurs, les groupes d'anneaux étant au nombre de k, le procédé comprenant les étapes suivantes:
a) pour chaque cycle (100, 110, 200, 210, 300, 310) d'une pluralité de cycles comprenant chacun k itérations d'émission-réception (101, 102, 103, 104, 105, 111, 112, 113, 201, 202, 203, 211, 212, 213, 301, 302, 311, 312), chaque itération d'émission-réception faisant intervenir un groupe d'anneaux transducteurs différent, chaque cycle parcourant l'ensemble des k groupes d'anneaux transducteurs, lors de chaque itération d'émission-réception dudit cycle :
- a1) excitation d'un groupe d'anneaux transducteurs de sorte que les anneaux transducteurs dudit groupe d'anneaux transducteurs émettent des ondes ultrasonores à une fréquence d'émission ;
- a2) récupération de n signaux de mesure des n anneaux transducteurs, chaque signal de mesure résultant de la réception par un anneau transducteur d'ondes ultrasonores réfléchies résultant de l'émission d'ondes ultrasonores par ledit groupe d'anneaux transducteurs excité lors de ladite itération d'émission-réception ;
- a3) combinaison des n signaux de mesure récupérés pour donner une ligne échographique pour ladite itération d'émission-réception, ladite ligne échographique étant représentative de la réponse des n anneaux transducteurs à l'émission d'ondes ultrasonores par ledit groupe d'anneaux ultrasonore excité lors de ladite itération d'émission-réception ;
b) parallèlement à la poursuite des cycles suivant le premier cycle, combinaison (106, 107, 108, 204, 205, 206, 303, 304, 305) en une ligne affichable de k lignes échographiques résultant des k itérations d'émission-réception les plus récentes lorsque lesdites lignes échographiques sont disponibles ;
c) traitement et affichage des lignes affichables.

2. Procédé selon la revendication précédente, dans lequel dans chaque cycle, les itérations d'émission-réception sont effectuées dans le même ordre.

3. Procédé selon l'une quelconque des revendications précédentes, dans lequel le signal de mesure d'un anneau transducteur résulte de la numérisation du signal de réception généré par cet anneau transducteur lors de la réception par ledit anneau transducteur d'ondes ultrasonores réfléchies résultant de l'émission d'ondes ultrasonores par un groupe d'anneaux transducteurs, un signal de mesure se définissant comme une suite chronologique de points discrets auxquels sont associées des valeurs correspondantes, et dans lequel la combinaison de signaux de mesure pour donner une ligne échographique lors d'une itération d'émission-réception consiste à additionner les valeurs associées à des points discrets synchrones desdits signaux de mesure.

4. Procédé selon la revendication précédente, dans lequel la combinaison de signaux de mesure pour donner une ligne échographique lors d'une itération d'émission-réception est restreinte à une sélection de points discrets, lesdits points discrets étant sélectionnés de sorte à opérer un décalage chronologique entre les signaux de mesure compensant les différences de chemin acoustique résultant de la géométrie du transducteur, le synchronisme des points discrets prenant en compte ce décalage.

5. Procédé selon l'une des deux revendications précédentes, dans lequel des points discrets sont ajoutés au signal de mesure en faisant la convolution dudit signal de mesure avec un sinus cardinal glissant de sorte qu'une période entre les points discrets soit inférieure à l'inverse d'au moins dix fois la fréquence d'émission.

6. Procédé selon l'une des revendications précédentes, dans lequel une ligne échographique se définit comme une suite chronologique de points discrets auxquels sont associées des valeurs correspondantes, et dans lequel la combinaison de lignes échographiques pour donner une ligne affichable consiste à additionner les valeurs associées à des points discrets synchrones desdites lignes échographiques.

7. Procédé selon l'une quelconque des revendications précédentes, dans lequel les anneaux transducteurs sont regroupés en k groupes d'anneaux regroupant chacun entre 2 et n-1 anneaux transducteurs.

8. Procédé selon l'une quelconque des revendications précédentes, dans lequel n= 5, la sonde ultrasonore (1) comprenant cinq anneaux transducteurs (2).

9. Produit programme d'ordinateur comprenant des instructions de code de programme enregistrées sur un support non-volatile utilisable dans un ordinateur pour l'exécution des étapes de traitement du procédé selon l'une des revendications précédentes, lorsque ledit programme est exécuté sur un système selon la revendication 10.

10. Système d'échographie comprenant une sonde ultrasonore (1) comportant une pluralité d'éléments transducteurs organisés en au moins n anneaux concentriques formant n anneaux transducteurs (2, 2a, 2b, 2c, 2d, 2e), une unité de traitement de ladite sonde ultrasonore et un écran, ladite unité de traitement étant configurée pour mettre en œuvre le procédé selon l'une quelconque des revendications précédentes, les anneaux transducteurs étant regroupés en plusieurs groupes d'anneaux regroupant chacun entre 1 et n-1 anneaux transducteurs, chaque groupe d'anneaux se différenciant d'un autre groupe d'anneaux transducteurs par au moins un anneau transducteur différent des anneaux transducteurs dudit autre groupe d'anneaux transducteurs, les groupes d'anneaux étant au nombre de k, l'unité de traitement étant configurée pour :
a) pour chaque cycle d'une pluralité de cycles comprenant chacun k itérations d'émission-réception, chaque itération d'émission-réception faisant intervenir un groupe d'anneaux transducteurs différent, chaque cycle parcourant l'ensemble des k groupes d'anneaux transducteurs, lors de chaque itération d'émission-réception :
a1) exciter un groupe d'anneaux transducteurs de sorte que les anneaux transducteurs dudit groupe d'anneaux transducteurs émettent des ondes ultrasonores à une fréquence d'émission ;
a2) récupérer n signaux de mesure des n anneaux transducteurs, chaque signal de mesure résultant de la réception par un anneau transducteur d'ondes ultrasonores réfléchies résultant de l'émission d'ondes ultrasonores par ledit groupe d'anneaux transducteurs excité lors de ladite itération d'émission-réception;
a3) combiner les n signaux de mesure pour donner une ligne échographique, ladite ligne échographique étant représentative de la réponse des n anneaux transducteurs à l'émission d'ondes ultrasonores par ledit groupe d'anneaux ultrasonore excité lors de ladite itération d'émission-réception;
b) parallèlement à la poursuite des cycles suivant le premier cycle, combiner en une ligne affichable de k lignes échographiques résultant des k itérations d'émission-réception les plus récentes lorsque lesdites lignes échographiques sont disponibles ;
c) traiter et envoyer à l'écran les lignes affichables.

## Patentansprüche

1. Verfahren für Augen-Ultraschall, bei dem eine Ultraschallsonde (1) verwendet wird, die eine Vielzahl von Wandlerelementen aufweist, die in mindestens n konzentrischen Ringen organisiert sind, die n Wandlerringe (2, 2a, 2b, 2c, 2d, 2e) bilden, wobei die Wandlerringe in mehreren Ringgruppen vereint sind, die jeweils zwischen 1 und n-1 Wandlerringe vereinen, wobei sich jede Ringgruppe von einer anderen Wandlerringgruppe durch mindestens einen Wandlerring unterscheidet, der sich von den Wandlerringen der anderen Wandlerringgruppe unterscheidet, wobei die Anzahl der Ringgruppen k beträgt, wobei das Verfahren die folgenden Schritte umfasst:
a) für jeden Zyklus (100, 110, 200, 210, 300, 310) einer Vielzahl von Zyklen, die jeweils k Sende-Empfangs-Iterationen (101, 102, 103, 104, 105, 111, 112, 113, 201, 202, 203, 211, 212, 213, 301, 302, 311, 312) umfassen, wobei jede Sende-Empfangs-Iteration eine unterschiedliche Wandlerringgruppe einbezieht, wobei jeder Zyklus bei jeder Sende-Empfangs-Iteration des Zyklus alle k Wandlerringgruppen durchläuft:
- a1) Erregen einer Wandlerringgruppe, so dass die Wandlerringe der Wandlerringgruppe Ultraschallwellen mit einer Sendefrequenz senden
- a2) Abrufen von n Messsignalen von den n Wandlerringen, wobei jedes Messsignal aus dem Empfang von reflektierten Ultraschallwellen, die aus dem Senden von Ultraschallwellen durch die während der Sende-Empfangs-Iteration angeregte Wandlerringgruppe resultieren, durch einen Wandlerring resultiert;
- a3) Kombinieren der n abgerufenen Messsignale, um eine Ultraschall-Linie für die Sende-Empfangs-Iteration zu ergeben, wobei die Ultraschall-Linie für die Antwort der n Wandlerringe auf die Sendung von Ultraschallwellen durch die angeregte Ultraschallringgruppe während der Sende-Empfangs-Iteration repräsentativ ist;
b) parallel zur Fortsetzung der Zyklen nach dem ersten Zyklus, Kombination (106, 107, 108, 204, 205, 206, 303, 304, 305) von k Ultraschall-Linien, die sich aus den k jüngsten Sende-Empfangs-Iterationen ergeben, wenn diese Ultraschall-Linien verfügbar sind, in einer anzeigbaren Linie;
c) Verarbeitung und Anzeige der anzeigbaren Linien.

2. Verfahren nach vorhergehendem Anspruch, wobei die Sende-Empfangs-Iterationen in jedem Zyklus in der gleichen Reihenfolge durchgeführt werden.

3. Verfahren nach einem der vorhergehenden Ansprüche, wobei das Messsignal eines Wandlerrings aus der Digitalisierung des Empfangssignals resultiert, das von diesem Wandlerring beim Empfang reflektierter Ultraschallwellen, die aus der Sendung von Ultraschallwellen durch eine Wandlerringgruppe resultieren, durch den Wandlerring erzeugt wird, wobei ein Messsignal als eine chronologische Folge diskreter Punkte definiert ist, denen entsprechende Werte zugeordnet sind, und wobei die Kombination von Messsignalen zwecks Bildung einer Ultraschall-Linie während einer Sende-Empfangs-Iteration darin besteht, die Werte zu addieren, die synchronen diskreten Punkten der Messsignale zugeordnet sind.

4. Verfahren nach vorhergehendem Anspruch, wobei die Kombination von Messsignalen zwecks Bildung einer Ultraschall-Linie während einer Sende-Empfangs-Iteration auf eine Auswahl diskreter Punkte beschränkt ist, wobei die diskreten Punkte derart ausgewählt werden, dass ein zeitlicher Versatz zwischen den Messsignalen entsteht, der die Unterschiede im akustischen Pfad ausgleicht, die sich aus der Geometrie des Wandlers ergeben, wobei die Synchronität der diskreten Punkte diesen Versatz berücksichtigt.

5. Verfahren nach einem der beiden vorhergehenden Ansprüche, wobei dem Messsignal durch Faltung des Messsignals mit einem gleitenden Kardinalsinus diskrete Punkte hinzugefügt werden, so dass eine Periode zwischen den diskreten Punkten kleiner ist als der Kehrwert von mindestens dem Zehnfachen der Sendefrequenz.

6. Verfahren nach einem der vorhergehenden Ansprüche, wobei eine Ultraschall-Linie als eine chronologische Folge diskreter Punkte definiert ist, denen entsprechende Werte zugeordnet sind, und wobei die Kombination von Ultraschall-Linien, um eine anzeigbare Linie zu erhalten, darin besteht, die Werte zu addieren, die synchronen diskreten Punkten der Ultraschall-Linien zugeordnet sind.

7. Verfahren nach einem der vorhergehenden Ansprüche, wobei die Wandlerringe in k Ringgruppen zusammengefasst werden, die jeweils zwischen 2 und n-1 Wandlerringe vereinen.

8. Verfahren nach einem der vorhergehenden Ansprüche, wobei n=5, wobei die Ultraschallsonde (1) fünf Wandlerringe (2) umfasst.

9. Rechnerprogrammprodukt, das Programmcodeanweisungen umfasst, die auf einem nichtflüchtigen Medium gespeichert sind, das in einem Rechner zur Ausführung der Verarbeitungsschritte des Verfahrens nach einem der vorhergehenden Ansprüche verwendbar ist, wenn das Programm auf einem System nach Anspruch 10 ausgeführt wird.

10. Ultraschallsystem, das eine Ultraschallsonde (1) umfasst, die eine Vielzahl von Wandlerelementen, die in mindestens n konzentrischen Ringen organisiert sind, die n Wandlerringe (2, 2a, 2b, 2c, 2d, 2e) bilden, eine Verarbeitungseinheit der Ultraschallsonde und einen Bildschirm aufweist, wobei die Verarbeitungseinheit ausgelegt ist, um das Verfahren nach einem der vorhergehenden Ansprüche durchzuführen, wobei die Wandlerringe in mehreren Ringgruppen vereint sind, die jeweils zwischen 1 und n-1 Wandlerringe vereinen, wobei sich jede Ringgruppe von einer anderen Wandlerringgruppe durch mindestens einen Wandlerring unterscheidet, der sich von den Wandlerringen der anderen Wandlerringgruppe unterscheidet, wobei die Anzahl der Ringgruppen k beträgt, wobei die Verarbeitungseinheit ausgelegt ist, um:
a) für jeden Zyklus einer Vielzahl von Zyklen, die jeweils k Sende-Empfangs-Iterationen umfassen, wobei jede Sende-Empfangs-Iteration eine unterschiedliche Wandlerringgruppe einbezieht, wobei jeder Zyklus bei jeder Sende-Empfangs-Iteration alle k Wandlerringgruppen durchläuft:
a1) eine Wandlerringgruppe derart zu erregen, dass die Wandlerringe der Wandlerringgruppe Ultraschallwellen mit einer Sendefrequenz senden;
a2) n Messsignale von den n Wandlerringen abzurufen, wobei jedes Messsignal aus dem Empfang von reflektierten Ultraschallwellen, die aus dem Senden von Ultraschallwellen durch die während der Sende-Empfangs-Iteration angeregte Wandlerringgruppe resultieren, durch einen Wandlerring resultiert;
a3) die n Messsignale zu kombinieren, um eine Ultraschall-Linie für die Sende-Empfangs-Iteration zu ergeben, wobei die Ultraschall-Linie für die Antwort der n Wandlerringe auf die Sendung von Ultraschallwellen durch die angeregte Ultraschallringgruppe während der Sende-Empfangs-Iteration repräsentativ ist;
b) parallel zur Fortsetzung der Zyklen nach dem ersten Zyklus k Ultraschall-Linien, die sich aus den k jüngsten Sende-Empfangs-Iterationen ergeben, in einer anzeigbaren Linie zu kombinieren, wenn diese Ultraschall-Linien verfügbar sind;
c) die anzeigbaren Linien zu verarbeiten und an den Bildschirm zu senden.

## Claims

1. Ocular echography method using an ultrasound probe (1) comprising a plurality of transducer elements organized in at least n concentric rings forming n transducer rings (2, 2a, 2b, 2c, 2d, 2e), in which the transducer rings are grouped into several ring groups each grouping between 1 and n-1 transducer rings, each ring group differing from another transducer ring group by at least one transducer ring different from the transducer rings of said other transducer ring group, the ring groups being k in number, the method comprising the following steps:
a) for each cycle (100, 110, 200, 210, 300, 310) of a plurality of cycles each comprising k transmit-receive iterations (101, 102, 103, 104, 105, 111, 112, 113, 201, 202, 203, 211, 212, 213, 301, 302, 311, 312), each transmit-receive iteration involving a different group of transducer rings, each cycle traversing all k groups of transducer rings, during each transmit-receive iteration of said cycle :
- a1) excitation of a group of transducer rings so that the transducer rings of said group of transducer rings emit ultrasonic waves at an emission frequency
- a2) recovery of n measurement signals from the n transducer rings, each measurement signal resulting from the reception by a transducer ring of reflected ultrasonic waves resulting from the transmission of ultrasonic waves by said group of transducer rings excited during said transmission-reception iteration ;
- a3) combination of the n measurement signals recovered to give an echographic line for said transmit-receive iteration , said echographic line being representative of the response of the n transducer rings to the emission of ultrasonic waves by said group of ultrasonic rings excited during said transmit-receive iteration;
b) simultaneously to the continuation of the cycles following the first cycle, combination (106, 107, 108, 204, 205, 206, 303, 304, 305) into a displayable line of k ultrasound lines resulting from the k most recent transmit-receive iterations when said ultrasound lines are available ;
c) processing and display of displayable lines.

2. Method according to the preceding claim, in which in each cycle, the transmit-receive iterations are carried out in the same order.

3. Method according to any one of the preceding claims, in which the measurement signal of a transducer ring results from the digitization of the reception signal generated by this transducer ring upon reception by said transducer ring of reflected ultrasonic waves resulting from the emission of ultrasonic waves by a group of transducer rings, a measurement signal being defined as a chronological sequence of discrete points to which corresponding values are associated,
and in which the combination of measurement signals to give an echographic line during a transmit-receive iteration consists of summing the values associated with synchronous discrete points of said measurement signals.

4. A method according to the preceding claim, in which the combination of measurement signals to give an echographic line during a transmission-reception iteration is restricted to a selection of discrete points, said discrete points being selected so as to operate a chronological offset between the measurement signals compensating for the differences in acoustic path resulting from the geometry of the transducer, the synchronism of the discrete points taking this offset into account.

5. A method according to one of the two preceding claims, in which discrete points are added to the measurement signal by convolving said measurement signal with a sliding cardinal sine such that a period between the discrete points is less than the inverse of at least ten times the transmission frequency.

6. A method according to one of the preceding claims, in which an echographic line is defined as a chronological sequence of discrete points to which corresponding values are associated, and in which the combination of echographic lines to give a displayable line consists in summing the values associated with synchronous discrete points of said echographic lines.

7. Method according to any of the preceding claims, in which the transducer rings are grouped into k ring groups each comprising between 2 and n-1 transducer rings.

8. Method according to any of the preceding claims, in which n= 5, the ultrasonic probe (1) comprising five transducer rings (2).

9. A computer program product comprising program code instructions stored on a non-volatile medium usable in a computer for executing the processing steps of the method according to any of the preceding claims, when said program is executed on a system according to claim 10.

10. Echography system comprising an ultrasound probe (1) having a plurality of transducer elements organized in at least n concentric rings forming n transducer rings (2, 2a, 2b, 2c, 2d, 2e), a processing unit for said ultrasound probe and a screen, said processing unit being configured to implement the method according to any one of the preceding claims, the transducer rings being grouped into several ring groups each comprising between 1 and n-1 transducer rings, each ring group differing from another transducer ring group by at least one transducer ring different from the transducer rings of said other transducer ring group, the ring groups being k in number, the processing unit being configured to :
a) for each cycle of a plurality of cycles, each cycle comprising k transmit-receive iterations, each transmit-receive iteration involving a different group of transducer rings, each cycle traversing all k groups of transducer rings, during each transmit-receive iteration:
a1) exciting a group of transducer rings so that the transducer rings of said group of transducer rings emit ultrasonic waves at an emission frequency;
a2) recovering n measurement signals from the n transducer rings, each measurement signal resulting from the reception by a transducer ring of reflected ultrasonic waves resulting from the emission of ultrasonic waves by said group of transducer rings excited during said transmit-receive iteration;
a3) combining the n measurement signals to give an echographic line, said echographic line being representative of the response of the n transducer rings to the emission of ultrasonic waves by said group of excited ultrasonic rings during said transmit-receive iteration;
b) simultaneously to the continuation of the cycles following the first cycle, combining into a displayable line of k echographic lines resulting from the k most recent transmit-receive iterations when said echographic lines are available;
c) processing and sending displayable lines to the screen.
